# EUROPEAN PATENT APPLICATION

(11) **EP 4 324 470 A1**
(43) Date of publication of application: **21.02.2024**
(21) Application number: 22788357.6
(22) Date of filing: 08.04.2022
(51) Int. Cl.: A61K 35/44, A61K 35/28, A61P 9/00

(54) **COMPOSITION CONTAINING MESENCHYMAL STEM CELL AND VASCULAR ENDOTHELIAL PROGENITOR CELL AS ACTIVE INGREDIENT FOR PREVENTION OR TREATMENT OF OCCLUSIVE VASCULAR DISEASE OR COMPLICATION THEREOF**

(30) Priority: 16.04.2021 KR 20210049817
(71) Applicant: UNIVERSITY-INDUSTRY COOPERATION GROUP OF KYUNG HEE UNIVERSITY, Yongin-si, Gyeonggi-do 17104 (KR); Elphis Cell Therapeutics, Seoul 02455 (KR)
(72) Inventor: HONG, Hyun Sook, Seoul 05619 (KR); HWANG, Dae Yeon, Goyang-si Gyeonggi-do 10517 (KR); KIM, Suna, Yongin-si Gyeonggi-do 17106 (KR); SON, Youngsook, Seoul 06305 (KR)
(74) Representative: Isarpatent
(86) International application number: PCT/KR2022/005118
(87) International publication number: WO 2022/220501

(57) **Abstract**

The present invention relates to a composition comprising vascular endothelial progenitor cells and mesenchymal stem cells as active ingredients for prevention or treatment of occlusive vascular diseases or complications thereof. Since it was found that vascular endothelial progenitor cells and mesenchymal stem cells remarkably improved vascularization ability upon application thereof in combination, the present invention has the effect of providing a new cell therapy product for prevention or treatment of occlusive vascular diseases or ulcers resulting therefrom, and a preparation method therefore.

## Description

### [Technical Field]

The present invention relates to a composition for prevention or treatment of occlusive vascular diseases or complications thereof including mesenchymal stem cells and vascular endothelial progenitor cells as active ingredients.

### [Background Art]

Vascular endothelial progenitor cells (EPCs) are cells that were first identified in peripheral blood and stimulate angiogenesis.

New blood vessels are generated by angiogenesis, arteriogenesis and vasculogenesis, and the angiogenesis is related to the proliferation and migration of vascular endothelial cells that grow from already existing mature vascular endothelial cells. The arteriogenesis is a process of remodeling existing arteriolar connections into collateral vessels, and the vasculogenesis proceeds through a process in which vascular endothelial progenitor cells differentiate into mature vascular endothelial cells. Therefore, the vascular endothelial progenitor cells circulating from the bone marrow migrate to a vascular damage area, and are involved in neovascularization through direct insertion into newly formed blood vessels or secretion of various angiogenesis and trophic factors.

Therefore, the vascular endothelial progenitor cells are attracting attention as potential targets in the field of regenerative medicine and for therapeutic purposes through reangiogenesis. Accordingly, studies have been conducted to increase the function of the vascular endothelial progenitor cells, and for example, studies on the preparation of recombinant EPCs obtained by modifying *ex vivo* genes have been conducted, and a technique for increasing the pro-angiogenic capacity of EPCs using a vascular endothelial growth factor (VEGF) or hypoxiainducible factor-1α has been studied.

Meanwhile, two types of cells that are currently used as cell therapy in occlusive vascular diseases are divided. There are BM-mesenchymal stem cells (MSC) and adipocyte-derived stem cells (ADSC) in a paracrine effect method that indirectly helps in vascularization by secreting large amounts of G-CSF mobilized MNC and bone marrow (BM)-EPC that may directly differentiate into blood vessels in an ischemic niche and growth factors contributing to the angiopoiesis.

However, since these cell therapy methods are single cell administration, differentiation and functions into complex blood vessels are difficult, and there are limitations in that it is difficult to expect a therapeutic effect only with a paracrine effect without direct vascularization. In order to solve these vascular structural and functional problems, the role of smooth muscle cells is important for the regeneration of not only vascular endothelial cells but also pericytes, especially arterioles, but it is known that mesenchymal stem cells serve as these pericytes and smooth muscle cells to contribute to the stability and functions of the formed blood vessels.

Accordingly, the present inventors confirmed the formation of stable blood vessels and restoration of blood flow in lower limb arterial occlusive disease when vascular endothelial cells that may directly differentiate into vascular endothelial cells in an ischemic niche by a combined administration method rather than the existing single administration method and bone marrow-derived mesenchymal stem cells capable of serving as pericytes and smooth muscle cells were treated in combination, and completed the present invention.

### [Disclosure]

### [Technical Problem]

An object of the present invention is to provide a cell therapy product composition for prevention or treatment of occlusive vascular diseases or ulcers resulting therefrom, including vascular endothelial progenitor cells and mesenchymal stem cells as active ingredients.

Another object of the present invention is to provide a preparation method of a cell therapy product for prevention or treatment of occlusive vascular diseases or ulcers resulting therefrom.

Another object of the present invention is to provide a pharmaceutical composition for prevention or treatment of occlusive vascular diseases or ulcers resulting therefrom, including vascular endothelial progenitor cells and mesenchymal stem cells as active ingredients.

Another object of the present invention is to provide a method for preventing or treating occlusive vascular diseases or ulcers resulting therefrom.

### [Technical Solution]

In order to achieve the object of the present invention, the present invention provides a cell therapy product composition for prevention or treatment of occlusive vascular diseases or ulcers resulting therefrom, including vascular endothelial progenitor cells and mesenchymal stem cells as active ingredients.

Subsequently, the present invention provides a preparation method of a cell therapy product for prevention or treatment of occlusive vascular diseases or ulcers resulting therefrom, including mixing vascular endothelial progenitor cells and mesenchymal stem cells.

Furthermore, the present invention provides a pharmaceutical composition for prevention or treatment of occlusive vascular diseases or ulcers resulting therefrom, including vascular endothelial progenitor cells and mesenchymal stem cells as active ingredients.

Finally, the present invention provides a method for preventing or treating occlusive vascular diseases or ulcers resulting therefrom, including administering the pharmaceutical composition to a subject.

### [Advantageous Effects]

According to the present invention, since it was found that vascular endothelial progenitor cells and mesenchymal stem cells remarkably improved vascularization ability upon application thereof in combination, there is an effect of providing a new cell therapy product for prevention or treatment of occlusive vascular diseases or ulcers resulting therefrom, and a preparation method therefor.

### [Description of Drawings]

FIG. 1 is a photograph briefly showing a blood flow analysis experiment method.
FIG. 2 is a photograph briefly showing a distribution test method of DiR-EL-100 through a fluorescence image analyzer.
FIG. 3 is a photograph showing a result of observing vascularization ability according to a change in cell ratio.
FIG. 4 is a photograph showing a result of observing vascularization ability according to a culture period.
FIG. 5 is a photograph showing a result of a foot preservation experiment by recovery of blood flow by composite stem cells in an occlusive vascular disease animal model.
FIG. 6 is a photograph showing a result of observing vascularization ability *in vivo* by composite stem cells.
FIG. 7 is a photograph and a graph showing a result of confirming cell migration and survival *in vivo* of composite stem cells.
FIG. 8 is a photograph showing a result of observing *in-vivo* biodistribution in organs of composite stem cells.
FIG. 9 is a photograph showing a tissue analysis result after administration of composite stem cells.
FIG. 10 is a schematic view of an experiment for evaluating therapeutic efficacy for each dose of composite stem cells.
FIG. 11 is a photograph and a graph showing a result of observing external changes of surgical lesions for each administered dose of composite stem cells.
FIG. 12 is a photograph and a graph showing a blood flow analysis result for each administered dose of composite stem cells.
FIG. 13 is a photograph showing a result of observing vascularization ability of composite stem cells in ischemic tissue.

### [Best Mode for the Invention]

Hereinafter, an embodiment of the present invention will be described in detail with reference to the accompanying drawings. However, the following embodiments are presented as examples for the present invention, and when it is determined that a detailed description of well-known technologies or configurations known to those skilled in the art may unnecessarily obscure the gist of the present invention, the detailed description thereof may be omitted, and the present invention is not limited thereto. Various modifications and applications of the present invention are possible within the description of claims to be described below and the equivalent scope interpreted therefrom.

Terminologies used herein are terminologies used to properly express embodiments of the present invention, which may vary according to a user, an operator's intention, or customs in the art to which the present invention pertains. Accordingly, definitions of the terminologies need to be described based on contents throughout this specification. Throughout the specification, when a part "comprises" a certain component, it is meant that the part may further include other components, not excluding other components, unless explicitly described to the contrary.

Throughout this specification, `%' used to indicate the concentration of a specific material is solid/solid (w/w)%, solid/liquid (w/v)%, and liquid/liquid (v/v)%, unless otherwise stated.

The "thrombosis" of the present invention is aggregates of blood factors, mainly consists of aggregates of platelets and fibrin, and is usually formed to prevent excessive bleeding in blood vessels. When platelets come into contact with the surface of the subendothelium to generate thrombosis, a reaction for blocking a vascular system is initiated, this process is called hemostasis, and the process is very important to block excessive bleeding at the wound site. Arterial thrombosis causes severe disease by local occlusion, whereas venous thrombosis mainly causes distant occlusion, or embolism.

At this time, "occlusion" is a term encompassing a state in which blood vessels are narrowed due to complete or partial blockage of blood vessels. The degree of occlusion may be determined based on the measured blood flow. That is, the degree of occlusion is classified as partial occlusion or complete occlusion, and the partial occlusion means a case where the baseline blood flow is reduced to 50 to 60%, and the complete occlusion means a case where the baseline blood flow is reduced to 90 to 100%.

Two types of cells that are currently used as cell therapy in occlusive vascular diseases are divided. There are BM-mesenchymal stem cells (MSCs) and adipocyte-derived stem cells (ADSCs) in a paracrine effect method that indirectly helps in vascularization by secreting large amounts of G-CSF mobilized MNC and bone marrow (BM)-EPC that may directly differentiate into blood vessels in an ischemic niche and growth factors contributing to the angiopoiesis.

However, since these cell therapy methods are single cell administration, differentiation and functions into complex blood vessels are difficult, and there are limitations in that it is difficult to expect a therapeutic effect only with a paracrine effect without direct vascularization.

Accordingly, the present inventors confirmed the formation of stable blood vessels and restoration of blood flow in lower limb arterial occlusive disease when vascular endothelial cells that may directly differentiate into vascular endothelial cells in an ischemic niche by a combined administration method rather than the existing single administration method and bone marrow-derived mesenchymal stem cells capable of serving as pericytes and smooth muscle cells were treated in combination, and completed the present invention.

Accordingly, the present invention provides a cell therapy product composition for prevention or treatment of occlusive vascular diseases or ulcers resulting therefrom, including vascular endothelial progenitor cells and mesenchymal stem cells as active ingredients.

The term "mesenchymal stem cells (MSCs)" used herein refer to multipotent stem cells capable of differentiating into various mesenchymal cells including bone, cartilage, fat, and muscle cells. The mesenchymal stem cells may be derived from umbilical cord, cord blood, bone marrow, fat, muscle, nerve, skin, amnion, chorion, decidua or placenta, preferably human bone marrow-mesenchymal stem cells (BM-MSCs). The mesenchymal stem cells have the ability to regenerate damaged tissues and cells by migrating directly to a damaged area of the body and may easily proliferate *in vitro,* and have been used as useful targets in gene therapy and cell therapy as multipotent cells capable of differentiating into various cell forms.

Further, in one embodiment of the present invention, the vascular endothelial progenitor cells and the mesenchymal stem cells may be mixed at a ratio of 2 : 1, but are not limited thereto.

Further, in one embodiment of the present invention, the vascular endothelial progenitor cells and the mesenchymal stem cells may be human bone marrow-mesenchymal stem cells, but are not limited thereto.

In addition, in one embodiment of the present invention, the occlusive vascular disease may be a disease selected from the group consisting of cerebrovascular disease (CVD), cardiovascular disease, arteriovascular disease, coronary artery disease (CAD) and peripheral artery disease (PAD), preferably coronary artery disease (CAD) and peripheral artery disease (PAD). Specifically, the occlusive vascular diseases include stroke, cerebral infarction, cerebral thrombosis, cerebral embolism, lacunar infarction, acute coronary syndrome, angina pectoris, aortic stenosis, myocardial infarction, bundle-branch block, cerebral ischemia, acute ischemic arteriovascular event, thrombophlebitis, venous thromboembolism, deep vein thrombosis, pulmonary embolism, peripheral vascular disease, atherosclerosis, vasospasm, restenosis, vascular occlusion due to vasculitis, and the like.

As used herein, the term "cerebrovascular disease (CVD)" is an arteriovascular disease that occurs in blood vessels supplying oxygen-rich blood to the face and brain, and generally, includes ischemic diseases or diseases causing deficiency of blood flow as well as comorbid disease that occurs together with CAD and/or peripheral artery disease (PAD). For example, the CVD includes ischemic cerebrovascular disease, acute infarction, stroke, ischemic stroke, hemorrhagic stroke, varicose veins, mild cognitive impairment (MCI) or transient ischemic attacks (TIA), but is not limited thereto.

As used herein, the term "cardiovascular disease" or "arteriovascular disease" is a general term used to classify a number of conditions that affect the heart, heart valves, blood and vasculature of the body, and includes diseases affecting the heart or blood vessels. Preferably, the "cardiovascular disease" or "arteriovascular disease" includes metabolic syndrome, syndrome X, atherosclerosis, atherothrombosis, coronary artery disease, stable and unstable angina, stroke, aortic disease such as aortic stenosis or aortic aneurysm, cerebrovascular disease, peripheral vascular disease or acute ischemic atherosclerotic event, but is not limited thereto.

As used herein, the term "coronary artery disease (CAD)" refers to arteriovascular disease that occurs when the artery (coronary artery) supplying blood to the myocardium is hardened and/or narrowed by atherosclerotic and calcium precipitation. The CAD causes a decrease in blood flow to the myocardium, and as a result, the myocardium does not receive a sufficient amount of oxygen, thereby ultimately causing necrosis. The CAD includes acute coronary syndrome, myocardial infarction (heart attack), angina pectoris (stable and unstable) or atherosclerosis and atherothrombosis occurring in the blood vessels supplying the blood to the heart, but is not limited thereto.

As used herein, the term "peripheral artery disease (PAD)" refers to disease such as atherosclerosis and atherothrombosis that occurs areas other than the heart and brain, and includes comorbid disease that generally occurs with CAD.

The term "ulcer (ischaemic ulcer)" used herein refers to a condition in which tissue is lost beyond the mucosal muscle plate, and in the present invention, means stasis ulcer which is ulcer caused by venous ulcer or peripheral vascular disease (PVD), ischemic ulcer which is arterial ulcer, or neuropathic ulcer which is ulcer occurring commonly in diabetic patients.

In addition, the vascular endothelial progenitor cells may be located in vascular cells when administered to a subject.

In addition, the mesenchymal stem cells may be located in pericytes when administered to a subject.

In addition, the subject means a mammal that is a subject of treatment, observation or experiment, and preferably means a human.

The term "pericyte" used herein refers to a vascular mural cell located within the basal membrane of the microvascular system, which forms a specific local contact with the vascular endothelium. The pericytes are connective tissue cells around small blood vessels, also called Rouget cells, adventitial cells or mural cells, and known to surround 10% to 50% of the outside of the vascular endothelium. The pericyte is a thin and elongated contractile cell that surrounds around the precapillary arteriole outside the basal membrane.

The pericytes are relatively undifferentiated pluripotent cells that function to support blood vessels and may differentiate into fibroblasts, smooth muscle cells, or macrophages as needed. In addition, the pericytes play an important role in bloodbrain barrier stability as well as angiogenesis, and may regulate blood flow in the microvascular system by adhesion to intravascular cells.

Therefore, the cell therapy product according to the present invention may further improve angiogenesis, vasculogenesis, and stability of blood vessel barriers through combined administration of vascular endothelial progenitor cells and mesenchymal stem cells.

The term "cell therapy product" used herein refers to a drug (US FDA regulation) used for the purpose of treatment, diagnosis, and prevention by cells and tissues prepared through isolation, culture, and special manipulation from subjects. In addition, the cell therapy product means a drug used for the purpose of treatment, diagnosis, and prevention through a series of actions, such as proliferating and selecting living autologous, allogeneic, or heterogeneous cells *in vitro,* changing the biological characteristics of cells by other methods, or the like to restore the function of cells or tissues.

The administration route of the cell therapy product composition may be administered through parenteral routes as long as the composition reaches a target tissue. The parenteral administration may be selected from the group consisting of, for example, intraperitoneal administration, intravenous administration, intramuscular administration, subcutaneous administration, intradermal administration, topical administration, intranasal administration, intrapulmonary administration, and rectal administration, but is not limited thereto.

The cell therapy product composition of the present invention may be used unfrozen or frozen for subsequent use. If it is required to be frozen, a standard cryopreservative (e.g., DMSO, glycerol, Epilife^{®} cell freezing medium (Cascade Biologics)) may be added to a pre-freezing cell group.

In addition, the cell therapy product composition may be formulated and administered into a unit dosage type of pharmaceutical preparation suitable for administration into the body of a patient according to a general method in a pharmaceutical field, and the preparation includes an effective dose by one or several administrations. A formulation suitable for the purpose preferably includes injection agents such as an injection ampoule, infusion agents such as an infusion bag, spray agents such as an aerosol agent, and the like, as parenterally administered preparations. The injection ampoule may be mixed and prepared with an injection immediately before use and the injection may use physiological saline, glucose, mannitol, a ringer's solution, and the like. In addition, the infusion bag may be made of polyvinyl chloride or polyethylene, and may exemplify infusion bags manufactured by Baxter, Becton Dickinson, Medcep, National Hospital Products, and Terumo corporations.

The pharmaceutical preparation may further include one or more general pharmaceutically acceptable inert carriers, for example, a preservative, a soothing agent, a solubilizer, a stabilizer, or the like in the case of an injection agent, and a base, an excipient, a lubricant, a preservative, or the like in the case of a local administering agent.

In addition, the cell therapy product composition may further include a support for accommodating the cells, preferably a biodegradable support. The biodegradable support may be a hydrogel such as fibrin glue, hyaluronic acid, gelatin, collagen, alginic acid, cellulose, pectin, chitin, polyglycolic acid, or polylactic acid, but is not limited thereto.

In addition, the cell therapy product composition may further include a pharmaceutically acceptable carrier, and the pharmaceutically acceptable carrier may be used by mixing saline, sterile water, Ringer's solution, buffered saline, dextrose solution, maltodextrin solution, glycerol, ethanol, and at least one of these ingredients, and if necessary, general other additives such as an antioxidant, a buffer and a bacteriostat may be added.

The buffer may optionally be acetate, citrate, tartrate, lactate, succinate, or phosphate. The stabilizer may be mannitol, histidine, lysine, glycine, sucrose, fructose, trehalose, lactose or a mixture thereof. The isotonic agent may be glycerin, lactose, mannitol, dextrose, sodium chloride, sodium sulfate or sorbitol. The antioxidant may be acetone, sodium bisulfite, butylated hydroxyanisole, butylated hydroxytoluene, cysteine, cysteinate HCl, dithionite sodium, gentisic acid, gentisic acid ethanolamine, glutamate monosodium, formaldehyde sodium sulfoxylate, potassium metabisulfite, sodium metabisulfite, monothioglycerol, propyl gallate, sodium sulfite, sodium thioglycolate, or ascorbic acid. The bulking agent may be mannitol, glycine, lactose, sucrose, trehalose, dextrin, hydroxyethyl starch, ficol or gelatin.

The cell therapy product composition or pharmaceutical preparation of the present invention prepared as such may be administered together with other stem cells used for transplantation and other uses or in the form of a mixture with such stem cells using an administration method commonly used in the art. Preferably, the cell therapy product composition or pharmaceutical preparation can be engrafted or transplanted directly to a disease site of a patient to be treated or transplanted or injected directly to the abdominal cavity, but is not limited thereto. In addition, the administration can use both non-surgical administrations using a catheter and surgical administration such as injection or transplantation after incision of a disease site, but a non-surgical administration method using a catheter is more preferable. In addition, according to a general method, as parenteral administration, for example, transplantation by intravascular injection as a common method of hematopoietic stem cell transplantation is also possible in addition to direct administration to lesions.

In addition, the daily dose of the mixture of vascular endothelial progenitor cells and mesenchymal stem cells may be administered once or several times in 1.0 × 10¹ to 1.0 × 10¹⁰ cells/kg body weight, preferably 1.0 × 10⁵ to 1.0 × 10⁹ cells/kg body weight, more preferably 1.2 × 10⁵ to 1.2 × 10⁷ cells/kg body weight. However, it should be understood that an actual dosage of the active ingredient should be determined by various related actors, such as a disease to be treated, the severity of the disease, a route of administration, weight, age and sex of a patient, and the like. Accordingly, the dosage is not limited to the scope of the present invention in any way.

Subsequently, the present invention provides a preparation method of a cell therapy product for prevention or treatment of occlusive vascular diseases or ulcers resulting therefrom, including mixing vascular endothelial progenitor cells and mesenchymal stem cells.

Since the preparation method of the cell therapy product of the present invention includes the above-described cell therapy product, the duplicated descriptions with the above-described cell therapy product of the present invention will be omitted in order to avoid excessive complexity of the present specification.

Furthermore, the present invention provides a pharmaceutical composition for prevention or treatment of occlusive vascular diseases or ulcers resulting therefrom, including vascular endothelial progenitor cells and mesenchymal stem cells as active ingredients.

Since the pharmaceutical composition of the present invention includes the above-described vascular endothelial progenitor cells and mesenchymal stem cells, the duplicated descriptions with the above-described vascular endothelial progenitor cells and mesenchymal stem cells of the present invention will be omitted in order to avoid excessive complexity of the present specification.

The composition of the present invention may be administered as an individual therapeutic agent or in combination with other therapeutic agents, and may be administered sequentially or simultaneously with existing therapeutic agents, and may be administered singly or multiply. It is important to administer an amount capable of obtaining a maximum effect with a minimal amount without side effects by considering all the factors, which may be easily determined by those skilled in the art.

The composition according to the present invention may include a pharmaceutically effective dose of a mixture of vascular endothelial progenitor cells and mesenchymal stem cells alone or one or more pharmaceutically acceptable carriers, excipients or diluents. The pharmaceutically effective dose refers to an amount enough to prevent, improve, and treat symptoms of immune diseases. The "pharmaceutically acceptable" refers to a composition that is physiologically acceptable and does not cause an allergic reaction, such as gastrointestinal disorder, dizziness, etc., or a similar reaction thereto when administered to humans.

In addition, the composition including the pharmaceutically acceptable carrier may have various oral or parenteral formulations. When the composition is formulated, the formulations may be prepared by using diluents or excipients, such as a filler, an extender, a binder, a wetting agent, a disintegrating agent, a surfactant, etc., which are generally used. The carriers, excipients and diluents may be at least one selected from the group consisting of lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, physiological saline, methylhydroxybenzoate, propylhydroxybenzoate, talc, magnesium stearate and mineral oil, dextrin, calcium carbonate, propylene glycol, and liquid paraffin, but are not limited thereto, and may be used as all conventional carriers, excipients or diluents. The ingredients may be added independently or in combination with the mixture of vascular endothelial progenitor cells and mesenchymal stem cells, which are the active ingredients.

Solid formulations for oral administration may include tablets, pills, powders, granules, capsules, and the like, and the solid formulations may be prepared by mixing at least one excipient, for example, starch, calcium carbonate, sucrose or lactose, gelatin, and the like with at least one compound. Further, lubricants such as magnesium stearate and talc may also be used in addition to simple excipents. Liquid formulations for oral administration may correspond to a suspension, an oral liquid, an emulsion, a syrup, and the like, and may include various excipients, for example, a wetting agent, a sweetener, an aromatic agent, a preserving agent, and the like, in addition to water and liquid paraffin which are commonly used as simple diluents.

Formulations for parenteral administration include a sterile aqueous solution, a non-aqueous solution, a suspension, an emulsion, a lyophilizing agent, a suppository, and the like. As the non-aqueous solution and the suspension, propylene glycol, polyethylene glycol, vegetable oil such as olive oil, injectable ester such as ethyl oleate, and the like may be used. As a base of the suppository, witepsol, macrogol, Tween 61, cacao butter, laurinum, glycerol, gelatin, and the like may be used.

Further, the pharmaceutical composition of the present invention may have any one formulation selected from the group consisting of tablets, pills, powders, granules, capsules, suspensions, oral liquids, emulsions, syrups, sterilized aqueous solutions, non-aqueous solvents, suspensions, emulsions, lyophilized agents, and suppositories. As a base of the suppository, witepsol, macrogol, Tween 61, cacao butter, laurinum, glycerol, gelatin, and the like may be used.

In addition, the effective dose for the human body of the mixture of the vascular endothelial progenitor cells and the mesenchymal stem cells of the present invention may vary depending on the age, weight, and sex of a patient, a dosage form, a health condition and a disease degree, and generally about 0.01 to 1000 mg/kg/day, preferably 0.01 to 350 mg/kg/day. Based on an adult patient with a body weight of 60 kg, the effective dose may be generally 0.6 to 60000 mg/day, preferably 0.6 to 2100 mg/day, and may also be divided once or several times a day at regular intervals according to the judgment of a doctor or pharmacist.

Finally, the present invention provides a method for preventing or treating occlusive vascular diseases or ulcers resulting therefrom, including administering the pharmaceutical composition to a subject.

In the present invention, the term "subject" means a subject in need of a method for preventing, controlling or treating a disease, and may be used without limitation, such as humans, dogs, monkeys, cats, rodents, such as mice and genetically engineered mice. More specifically, the subject refers to mammals such as humans or nonhuman primates, mice, rats, dogs, cats, horses, and cows.

The pharmaceutical composition of the present invention may be administered in a therapeutically effective dose or a pharmaceutically effective dose.

In the present invention, the term "therapeutically effective dose" means an amount of a pharmaceutically acceptable salt of the composition effective for preventing or treating a target disease, and the therapeutically effective dose of the composition of the present invention may vary depending on many factors, such as a method of administration, a target site, the condition of a patient, and the like. Accordingly, when used in the human body, the dose should be determined as an appropriate amount in consideration of both safety and efficiency. It is also possible to estimate the amount used in humans from the effective dose determined through animal experiments. These matters to be considered when determining the effective dose are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The term 'pharmaceutically effective dose' used herein refers to an amount enough to treat a disease at a reasonable benefit/risk ratio applicable to medical treatment and not causing side effects. An effective dose level may be determined according to factors including the health condition of a patient, the type and severity of a disease, the activity of a drug, the sensitivity to a drug, an administration method, an administration time, an administration route, an excretion rate, duration of treatment, and drugs used in combination or simultaneously, and other factors well-known in the medical field. The composition of the present invention may be administered as an individual therapeutic agent or in combination with other therapeutic agents, and may be administered sequentially or simultaneously with existing therapeutic agents, and may be administered singly or multiply. It is important to administer an amount capable of obtaining a maximum effect with a minimal amount without side effects by considering all the factors, which may be easily determined by those skilled in the art.

### [Modes for the Invention]

Hereinafter, the present invention will be described in more detail through Examples. However, these Examples are more specifically illustrative of the present invention, and the scope of the present invention is not limited to these Examples.

### <Example 1> Experimental Materials and Methods

### 1-1. Cell culture

Human bone marrow-mononuclear cells (MNCs) were prepared, seeded in each cell selection medium, and bone marrow-vascular endothelial progenitor cells and mesenchymal stem cells were isolated. The human bone marrow-mononuclear cells (MNCs) were purchased from STEMCELL Technologies Inc (Vancouver, Canada) and used.

For EPC culture, the isolated BM-MNCs were cultured in an endothelial growth medium-2 (EGM-2, Lonza, Basel, Switzerland) in a 100 mm dish coated with type I collagen (CellMatrix, VA, USA). The medium was replaced daily until day 7 and then replaced every 3 days. The EPCs were subcultured at 70 to 80% confluency.

For MSC culture, the BM-MNCs were cultured in a mesenchymal stem cell growth medium (Stem MACS, Miltenyi) in a 100 mm dish. The medium was replaced every 3 days and subcultured at 70 to 80% confluency.

### 1-2. Preparation of animal model

A Balb/c-nu mouse was anesthetized by intraperitoneal administration of Ketamin (100 mg/Kg) and Rompun (5 mg/kg), and then laid down in a supine position, and the leg was fixed using a medical tape and then the skin was incised from the ankle to the thigh to expose the common femoral artery. Thereafter, the total common femoral artery was ligated using a needle holder and suture 6-0 silk to block blood flow. The proximal region of the superficial femoral artery was ligated using 6-0 silk. At the time of ligation, since the superficial femoral artery and the nerve were very adjacent to each other, the superficial femoral artery and the nerve were separated using a micro-fine forcep, and then a suture needle was carefully inserted and then ligated. The distal region of the superficial femoral artery was ligated using 6-0 silk.

The distal ligation region of the superficial femoral artery was grasped using micro-fine forceps, carefully separated from the nerve, and lifted, and then the blood vessel was removed using surgical scissors. After the incised skin was sutured with 5-0 silk, blood flow was measured to determine whether ischemia appeared. Thereafter, the suture region was disinfected with povidone.

The administration method was performed by dividing the ischemic region (IR) muscle area near the blood vessel removed from the superficial femoral artery into 5 yellow spots at intervals of 2.0 mm to 2.5 mm using an insulin syringe (1 mL, 29 G) and administering 10 µL of povidone per spot and a total of 50 µL, in a single injection.

### 1-3. Immunofluorescent Staining

A dried slide was deparaffinized with xylene, and then washed from highconcentration alcohol to low-concentration alcohol. The tissue slide was immersed in 0.01 M citric acid (pH 6.0) as a buffer solution, heated in a microwave oven for 10 minutes, sufficiently cooled at room temperature, and then washed with PBS. In order to prevent autofluorescence of tissue, the tissue slide was immersed in 0.1% sodium borohydride (in PBS) at room temperature for 1 hour while maintaining shading, and washed thoroughly with PBS. For antibody penetration, the tissue slide was immersed in 0.1% Triton-X for 10 minutes and then washed. A blocking solution was made with 2% NHS, and then treated for 1 hour at room temperature.

Then, a diluted primary antibody (primary Ab) was treated. The tissue slide was stored at a temperature of 4°C overnight in a shaded state, and careful not to dry out. The tissue slide was sufficiently washed with PBS, and then treated with a secondary antibody (Second Ab) at room temperature for 1 hour. WGA was diluted in a 2% NHS blocking solution at a concentration of 5 µg/mL and then treated at 37°C for 1 hour, and DAPI was diluted in DW at a concentration of 10 to 20 µ/mL and then treated at room temperature for 5 minutes.

### 1-4. Blood flow analysis (Doppler test)

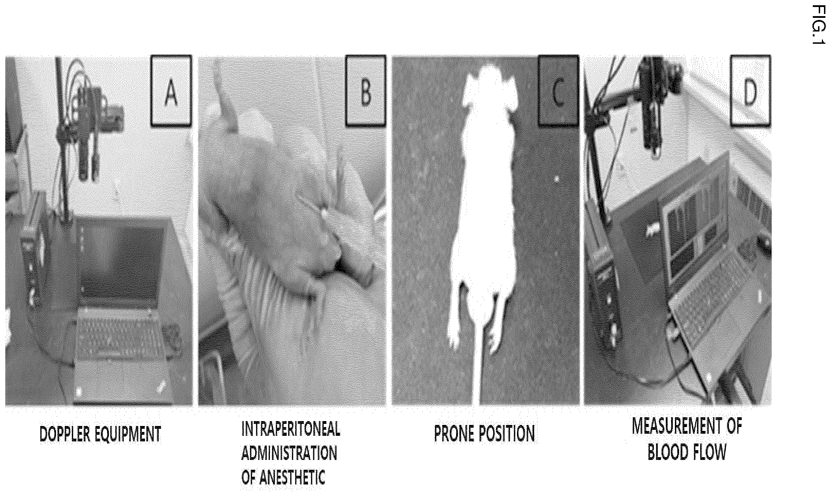

After installing laser doppler equipment and operating a device (FIG. 1A), the abdominal region of a mouse animal model was disinfected with an alcohol swab, and anesthetized by intraperitoneal injection of ketamine and rompun (FIG. 1B). Thereafter, the mouse animal model was laid in a prone position, and then blood flow was measured using the laser doppler equipment (FIGS. 1C and 1D).

### 1-5. Tube formation assay

Matrigel (Corning Inc, NY, USA) was treated to a µ-slide (Ibidi, Grafelfing, Germany) at 10 µl per well. Mixed cells of EPCs and MSCs were prepared at various ratios. The mixed cells were suspended in α-MEM (GIBCO, NY, USA) medium containing 0.2% FBS and seeded on the Matrigel, and then images were obtained with a microscope (Leica).

### 1-6. In vivo imaging

A distribution test of DiR-EL-100 through a fluorescence image analyzer (Maestro II, Whole Body Image) was performed in the following method.

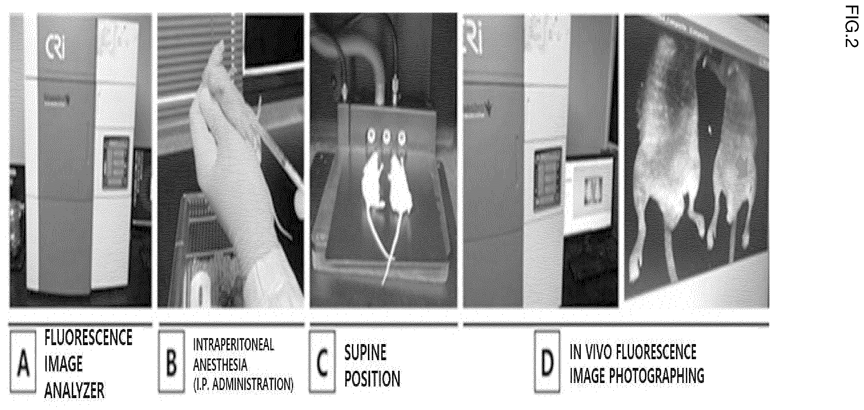

The power of the fluorescence image analyzer (Maestro II) and Maestro software on a computer connected to the device were turned on and waited for about 1 minute until initialization (FIG. 2A).

The abdominal region was disinfected with an alcohol swab, and then anesthetized by intraperitoneal injection of ketamine and rompun. After opening the door of the device, the anesthetized mouse was laid in a supine position and then the door was closed (FIGS. 2B and 2C). After pressing an interior illumination of the device, the location of the mouse and the photographing area were set. After an appropriate filter was selected, an optimal condition was set by setting a photographing size, exposure sensitivity, and the like (FIG. 2D).

After sacrifice, an autopsy was performed after photographing with an *in-vivo* fluorescence image analyzer (Maestro II). The abdominal cavity was incised and the abdominal vena cava and posterior vena cava were cut to be bled. Major organs/tissues (administration sites, such as femoral muscle, liver, spleen, brain, kidney, heart, lung, mesenteric lymph node, and femoral muscle on the opposite side of the administration sites) were extracted and washed thoroughly with cold PBS. The extracted organs/tissues are photographed with a fluorescence image analyzer (Maestro II).

### 1-7. Statistical analysis

All data were expressed as mean ± standard deviations, and a P value < 0.05 was considered as a significant value (*p < 0.05, **p < 0.01, ***p < 0.001).

### <Example 2> Analysis of vascularization ability according to ratio and culture period of composite stem cells

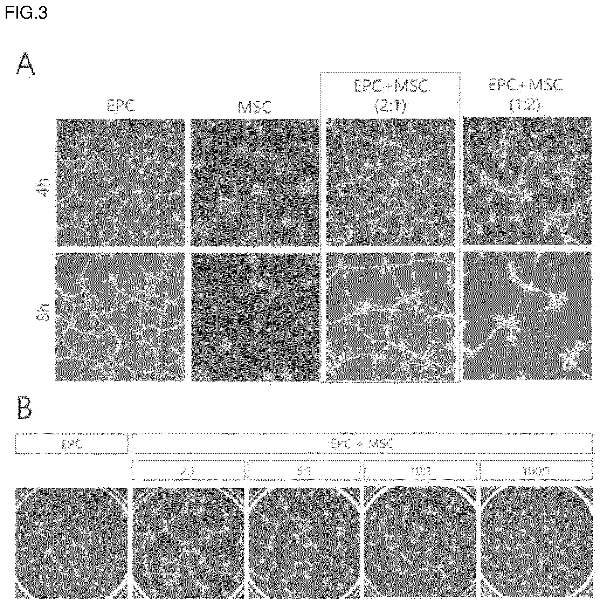

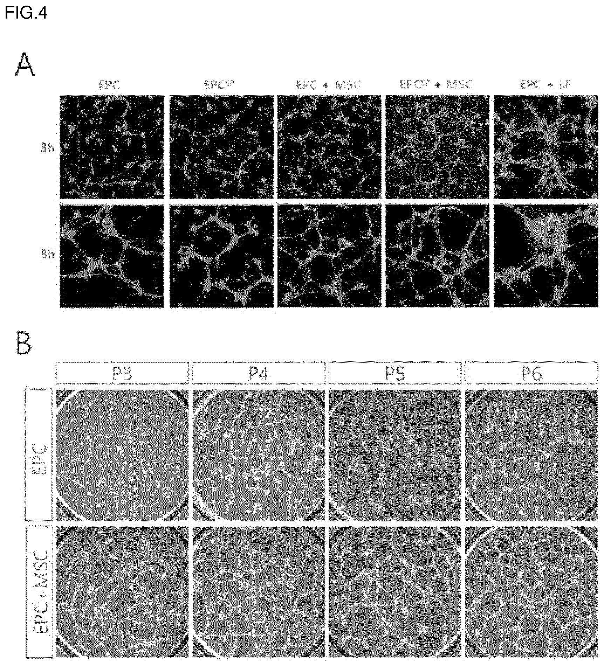

To confirm a change in vascularization ability according to a ratio and a culture period of vascular endothelial progenitor cells and mesenchymal stem cells, the vascular endothelial progenitor cells and the mesenchymal stem cells were mixed at ratios of 0 : 1, 1 : 0, 2 : 1, 1 : 2, 5 : 1, 10 : 1 and 100 : 1 to prepare composite stem cells, and each experimental group having the same number of cells was treated to observe the vascularization. The results were illustrated in FIGS. 3 and 4.

As a result of confirming the vascularization ability by cell ratio, the vascularization ability was observed even in an EPC-alone-treated group (1 : 0), but excellent vascularization ability was observed in a composite stem cell-treated group in which vascular endothelial progenitor cells and mesenchymal stem cells were mixed. In particular, in the case of composite stem cells in which vascular endothelial progenitor cells and mesenchymal stem cells were mixed at a ratio of 2 : 1, compared to the composite stem cells mixed at ratios of 1 : 2, 5 : 1, 10 : 1, and 100 : 1, it was confirmed that the composite stem cells had significantly better vascularization ability (see FIG. 3).

In addition, as a result of confirming the vascularization ability for each culture period, in the case of the composite stem cell-treated group in which vascular endothelial progenitor cells and mesenchymal stem cells were mixed, sophisticated cell rearrangement was observed in a short time, but in the case of composite stem cells in which vascular endothelial progenitor cells and fibroblast (LF) were mixed, such cell rearrangement was not observed (see FIG. 4A). In addition, the vascularization ability was decreased according to the aging of the vascular endothelial progenitor cells, but it was confirmed that when the vascular endothelial progenitor cells and the mesenchymal stem cells were mixed, the vascularization ability decreased by the aging of the vascular endothelial progenitor cells were restored (see FIG. 4B).

These results mean that the mesenchymal stem cells significantly improve the vascularization ability of the vascular endothelial progenitor cells. In subsequent experiments, composite stem cells were used, in which vascular endothelial progenitor cells having the highest vascularization ability and mesenchymal stem cells were mixed at a ratio of 2 : 1.

### <Example 3> Analysis of efficacy and vascularization ability by composite stem cells in occlusive peripheral arterial disease

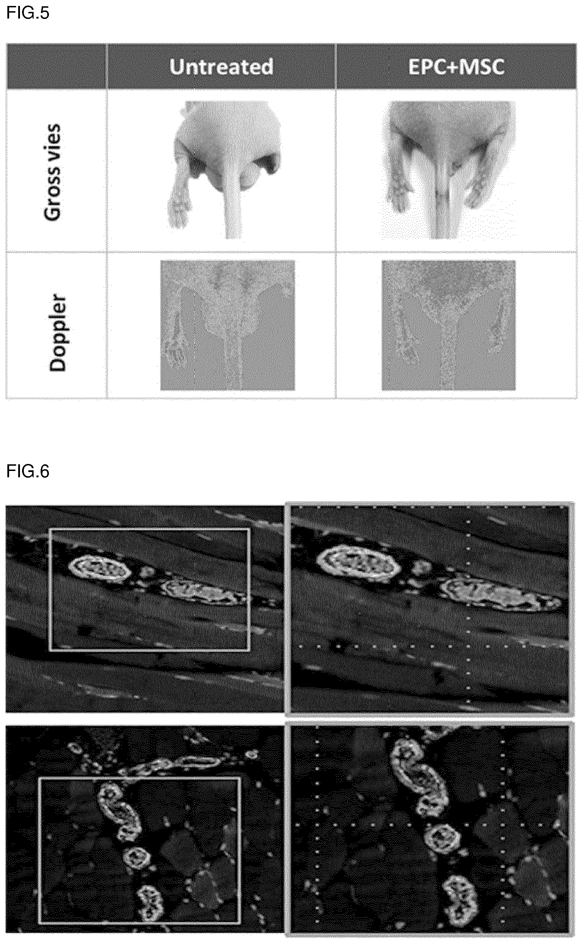

To confirm the efficacy and vascularization ability of composite stem cells in occlusive peripheral arterial disease, in a non-clinical animal model of lower limb arterial occlusive disease, it was confirmed whether the foot was preserved according to the recovery of blood flow and whether or not vascularization of composite stem cells *in vivo* was present. The results were illustrated in FIGS. 5 and 6.

As a result of confirming whether the foot was preserved according to the recovery of blood flow in the non-clinical animal model of lower limb arterial occlusive disease, foot preservation was not observed in the animal model to which the composite stem cells were not administered, but foot preservation was observed in the animal model to which the composite stem cells were administered (see FIG. 5). As a result of confirming the vascularization of composite stem cells *in vivo,* it was confirmed that the vascularization was present in the foot preserved by administration of the composite stem cells of the vascular endothelial progenitor cells and the mesenchymal stem cells *in vivo* (see FIG. 6).

### <Example 4> Observation of in-vivo distribution of composite stem cells after transplantation

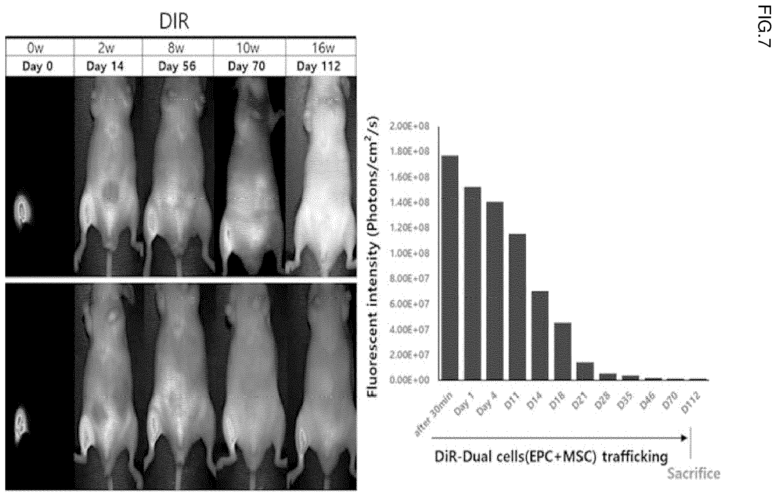

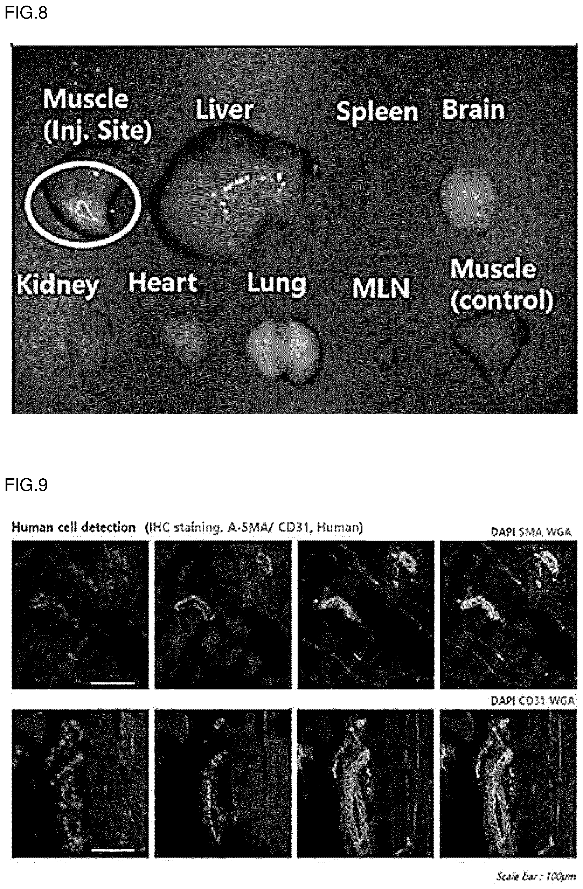

To observe *in-vivo* distribution of composite stem cells after transplantation, composite stem cells (EPC + MSC) were stained with DiR at a total dose of 1.2 × 10⁶/Mouse and then injected into the balb/c-nu femoral muscle, and thereafter, using an NIR wavelength of the fluorescence image analyzer (Maestro II), cell migration and survival *in vivo* were continuously observed until the fluorescence signal disappeared. In addition, after the fluorescence signal disappeared, an autopsy was conducted to confirm whether or not the administered composite stem cells remained in organs and tissues. The results were illustrated in FIGS. 7 and 8.

As illustrated in FIGS. 7 and 8, the intensity of fluorescence expression was most strongly measured after 30 minutes of the cell administration to the femoral muscle, and the fluorescence intensity was maintained at a high level until 2 weeks (14 days) and then gradually decreased until 3 weeks (21 days). In addition, after 4 weeks (28 days), similar fluorescence intensity was maintained until about 16 weeks (116 days), which means that the administered cells engrafted into the body. In addition, after 16 weeks (116 days), as a result of the autopsy of the mice administered with the composite stem cells, the fluorescence expression was detected only in the femoral muscle as the site of administration, and no fluorescence was observed in other organs and tissues. That is, it means that the composite stem cells transplanted into the body exist only at the site of administration and do not migrate to or remain in other tissues and organs.

Subsequently, histological analysis was performed to confirm what form each of the vascular endothelial progenitor cells and mesenchymal stem cells existed in the body, and the results were illustrated in FIG. 9. As illustrated in FIG. 9, the vascular endothelial progenitor cells (EPCs) were located in vascular cells, and the mesenchymal stem cells (MSCs) were located in pericytes. These results mean that the transplanted cells are maintained in the ischemic region and directly participate in angiogenesis in the host tissue.

### <Example 5> Evaluation of therapeutic efficacy for each dose of composite stem cells

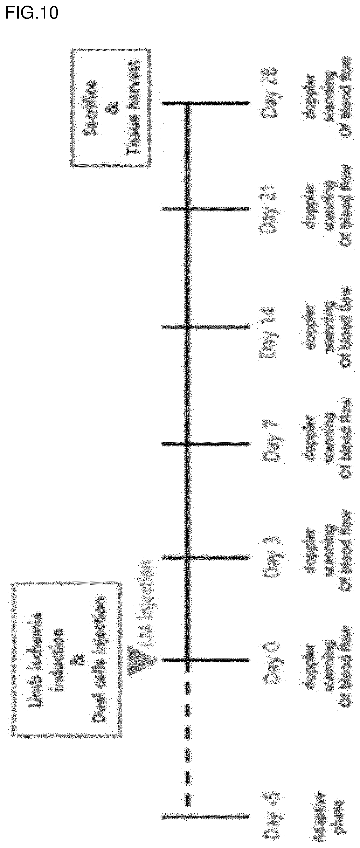

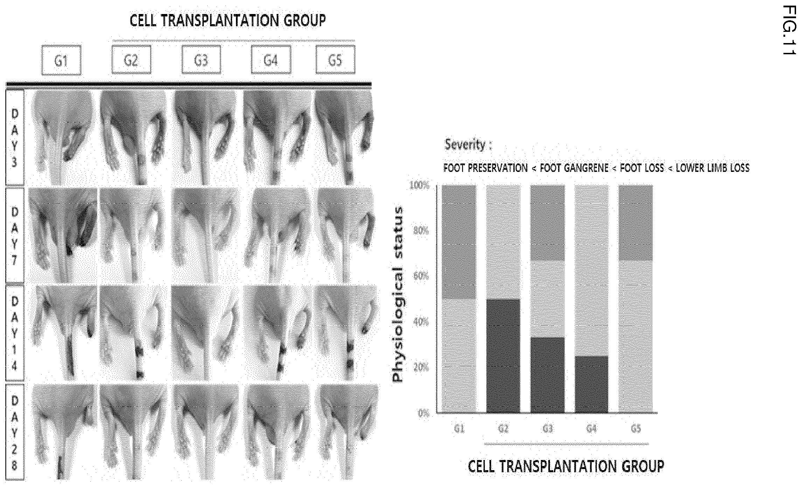

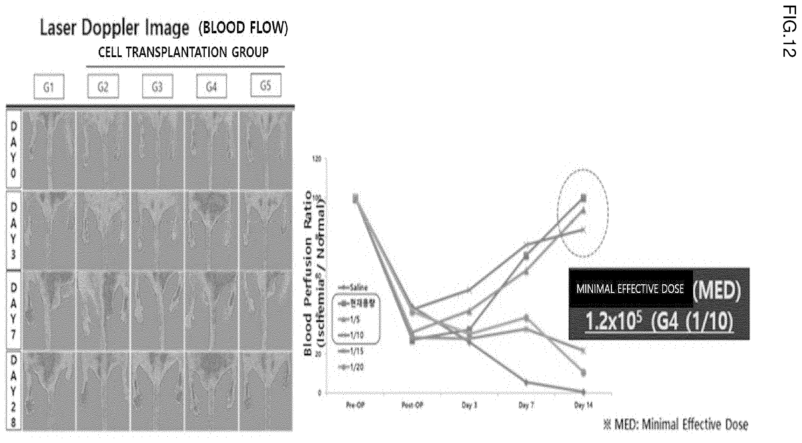

In order to evaluate the therapeutic efficacy of the composite stem cells (EPC + MSC) for each dose, an experiment for observing the external appearance of a surgical lesion and an experiment for measuring blood flow were performed for each cell dose (the number of transplanted cells). The experiments were designed with one control group and four experimental groups with the cell dose shown in Table 1 below, and the total volume was maintained the same, but the cells were transplanted from high dose to low dose and observed for 2 weeks (see FIG. 10). The results were illustrated in FIGS. 11 and 12.

**[Table 1]**

| Group | The number of transplanted cells (cell dose) | | | |
|---|---|---|---|---|
| | Dose range | EPC | MSC | Total |
| G1 | Saline | Saline | Saline | Saline |
| G2 | Reference (current) | 8.0 × 10⁵ | 4.0 × 10⁵ | 1. 2 × 10⁶ |
| G3 | 1/5 of G2 | 1.6 × 10⁵ | 8.0 × 10⁴ | 2.4 × 10⁵ |
| G4 | 1/10 of G2 | 8.0 × 10⁴ | 4.0 × 10⁴ | 1.2 × 10⁵ |
| G5 | 1/20 of G2 | 4.0 × 10⁴ | 2.0 × 10⁴ | 6.0 × 10⁴ |

As a result of observing the change in the appearance of the surgical lesion, the severity was very high in a control G1 group and a G5 group transplanted with a relatively small number of cells. On the other hand, in G2 to G4 groups transplanted with composite stem cells, all showed significant therapeutic effects (see FIG. 11). Through these results, it was confirmed that the effective range showing the therapeutic effect on occlusive vascular disease of composite stem cells was G2 (1.2 × 10⁶) to G4 (1.2 × 10⁵).

As a result of measuring the blood flow according to a cell dose, the control group G1 showed a decrease in blood flow and tissue loss over time. On the other hand, in the experimental group transplanted with the composite stem cells, it was confirmed that tissue preservation was observed as the blood flow was gradually restored (see FIG. 12A). In addition, as a result of measuring a blood perfusion ratio, it was observed that the blood flow was gradually improved and the tissue was recovered over time in the G2, G3, and G4 groups, and from these results, it can be seen that a minimum effective dose (MED) of composite stem cells is 1.2 × 10⁵.

### <Example 6> Confirmation of vascularization in ischemic tissue of composite stem cells

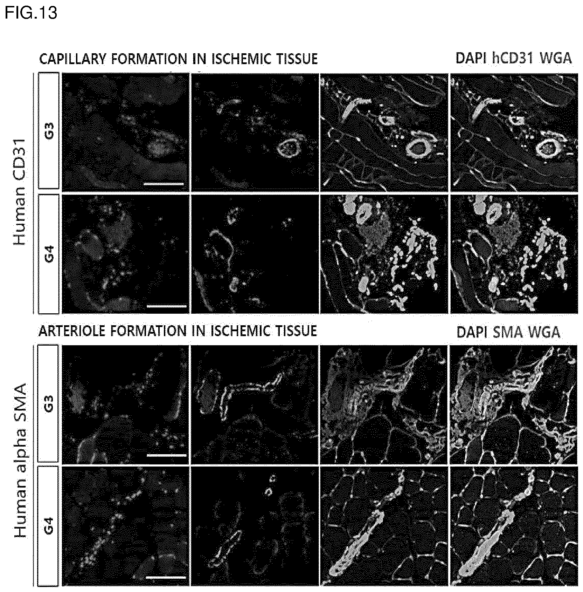

In order to confirm a relationship between vascularization and blood flow improvement in ischemic tissue of composite stem cells (EPC + MSC), tissue immunostaining was performed on the animal models of the G3 and G4 groups of Example 5. WGA was performed to confirm muscle and vascular structures, and the observed proteins confirmed vascular markers CD31 and alpha SMA. The results were shown in FIG. 13.

As illustrated in FIG. 13, it was confirmed that both G3 and G4 had vascularization in an ischemic environment, and no difference in vascularization was observed according to the number of cells.

## Claims

1. A cell therapy pharmaceutical composition prevention or treatment of occlusive vascular diseases or ulcers resulting therefrom, comprising vascular endothelial progenitor cells and mesenchymal stem cells as active ingredients.

2. The cell therapy pharmaceutical composition of claim 1, wherein the vascular endothelial progenitor cells and the mesenchymal stem cells are human bone marrow-mesenchymal stem cells.

3. The cell therapy pharmaceutical composition of claim 1, wherein the occlusive vascular disease is a disease selected from the group consisting of cerebrovascular disease (CVD), cardiovascular disease, arteriovascular disease, coronary artery disease (CAD) and peripheral artery disease (PAD).

4. The cell therapy pharmaceutical composition of claim 1, wherein the cell therapy product is administered at a dose of 1.2 × 10⁵ to 1.2 × 10⁷ cell/kg body weight.

5. The cell therapy pharmaceutical composition of claim 1, wherein the vascular endothelial progenitor cells are located in vascular cells when administered to a subject.

6. The cell therapy pharmaceutical composition of claim 1, wherein the mesenchymal stem cells are located in pericytes when administered to a subject.

7. A preparation method of a cell therapy product for prevention or treatment of occlusive vascular diseases or ulcers resulting therefrom, comprising mixing vascular endothelial progenitor cells and mesenchymal stem cells.

8. The preparation method of the cell therapy product of claim 7, wherein the vascular endothelial progenitor cells and the mesenchymal stem cells are mixed at a ratio of 2 : 1.

9. The preparation method of the cell therapy product of claim 7, wherein the vascular endothelial progenitor cells and the mesenchymal stem cells are human bone marrow-mesenchymal stem cells.

10. A pharmaceutical composition for prevention or treatment of occlusive vascular diseases or ulcers resulting therefrom, comprising vascular endothelial progenitor cells and mesenchymal stem cells as active ingredients.

11. A method for preventing or treating occlusive vascular diseases or ulcers resulting therefrom, comprising administering the pharmaceutical composition of claim 10 to a subject.
